# EUROPEAN PATENT APPLICATION

(11) **EP 4 667 571 A1**
(43) Date of publication of application: **24.12.2025**
(21) Application number: 24756898.3
(22) Date of filing: 14.02.2024
(51) Int. Cl.: C12N 15/113, A61K 31/7088, A61K 48/00, A61P 29/00, C40B 30/06

(54) **ARTIFICIAL MIRNA CONSTRUCT**

(30) Priority: 15.02.2023 JP 2023021890
(71) Applicant: INSTITUTE OF SCIENCE TOKYO, Tokyo 152-8550 (JP)
(72) Inventor: ASAHARA Hiroshi, Tokyo 152-8550 (JP); CHIBA Tomoki, Tokyo 152-8550 (JP)
(74) Representative: Plasseraud IP
(86) International application number: PCT/JP2024/004958
(87) International publication number: WO 2024/172057

(57) **Abstract**

One of the objects of the present invention is to provide an artificial miRNA construct useful for post-transcriptional mRNA regulation. The present invention uses a miRNA construct having a sequence, the sequence consisting of:
i) a seed sequence recognizing a binding motif of an RNA-binding protein;
ii) an adenine or uracil residue located on the 5' side of the seed sequence; and
iii) a scramble sequence located on the 3' side of the seed sequence, the scramble sequence having a sequence so that a GC content of the entire miRNA construct is 40-60%.

## Description

### [Technical Field]

The present invention relates to artificial miRNA constructs, pharmaceutical compositions comprising the same, and the uses thereof.

### [Background Art]

Chronic inflammation underlies the pathogenesis of many diseases, including age-related diseases such as osteoarthritis, autoimmune diseases such as rheumatoid arthritis, respiratory diseases such as asthma, allergic diseases such as atopic dermatitis, and metabolic syndromes such as diabetes. However, therapeutic agents for suppressing inflammation are limited to steroidal and non-steroidal anti-inflammatory drugs, and due to their insufficient effects on tissue-destructive inflammation as well as their side effects, the development of effective therapeutic methods based on elucidation of the molecular mechanisms underlying chronic inflammation is awaited.

Two factors involved in the onset and progression of such chronic inflammation are: cytokines such as TNF-α and IL-1β that directly elicit inflammation, and constitutive upregulation of matrix-degrading enzymes such as MMP-13 and ADAMTS5 that are involved in tissue damage. mRNA of these genes plays important roles in host defense responses and maintenance of homeostasis via acute inflammatory actions and tissue remodeling, but their expression is strictly regulated by not only the transcriptional level, but also mRNA stability at the post-transcriptional level and protein degradation so that it remains transient. Particularly, in post-transcriptional regulation of mRNA, two molecular mechanisms by RNA-binding proteins and microRNAs (miRNAs) regulate the transient expression of mRNA.

As RNA-binding proteins that suppress inflammatory cytokines, it was reported that Regnase-1 and Roquin recognize the specific recognition sites present in the 3' UTRs of mRNAs of inflammatory cytokines, IL-6 and TNF-α, respectively, and reduce mRNA stability to degrade mRNA but, as a converse result in genetic experiments in mice, that deletion of these factors results in delayed expression of target cytokines, to induce symptoms of autoimmune diseases (Non-Patent Documents 1 and 2).

In addition, the RNA-binding protein tristetraprolin (TTP) recognizes AU-rich elements (AREs) located in the 3' UTRs of cytokines and others, induces the CCR4-NOT complex to remove the poly(A) tail added to the 3' end of the mRNA. The deadenylated RNA is rapidly degraded by RNA-degrading enzymes. Mice lacking TTP systemically is known to exhibit a phenotype of inflammation due to constitutive expression of cytokines (Non-Patent Documents 3 and 4). Furthermore, although mice that lack TTP specifically in bone marrow cells do not show particular inflammatory symptoms under normal housing conditions, they exhibit excessive inflammatory responses to low LPS stimulation, compared to wild-type mice (Non-Patent Document 5).

miRNAs are single-stranded RNAs of about 21 to 25 nucleotides which are not translated into proteins. They are incorporated into the RNA-induced silencing complex (RISC), recognize the 3' UTRs of target genes, and suppress protein production by destabilizing the target mRNA or suppressing its translation. Notably, miR-146 specifically recognizes the 3' UTRs of mRNAs of inflammatory cytokines such as TNF-α and suppresses their expression. The present inventors have also reported that the expression of miR-146 correlates with the pathologies of rheumatoid arthritis and osteoarthritis (Non-Patent Document 6), and that knockout mice for miR-146a and miR-146b relate to the development of hematopoietic malignancies due to persistent chronic inflammation (Non-Patent Document 7).

These mRNA suppression mechanisms by RNA-binding proteins and miRNAs shorten the half-lives of mRNAs encoding inflammatory cytokines and tissue-degrading enzymes and thus they are only transiently expressed. However, it is considered that since these systems do not function adequately in the pathology of chronic inflammation, inflammation is sustained and disease onset and progression are involved. Therefore, the development of further therapeutic strategies capable of regulating the expression of target mRNAs is required.

### [Patent Documents]

[Non-Patent Document 1] Iwasaki H, et al., The IκB kinase complex regulates the stability of cytokine-encoding mRNA induced by TLR-IL-IR by controlling degradation of regnase-1, Nat Immunol., 12(12), 1167-75 (2011)
[Non-Patent Document 2] Yu D, et al., Roquin represses autoimmunity by limiting inducible T-cell co-stimulator messenger RNA, Nature., 450(7167), 299-303 (2007)
[Non-Patent Document 3] Carballo E, et al., Feedback Inhibition of Macrophage Tumor Necrosis Factor-a Production by Tristetraprolin, SCIENCE, 281(5379), 1001-1005 (1998)
[Non-Patent Document 4] Taylor GA, et al., A Pathogenetic Role for TNFα in the Syndrome of Cachexia, Arthritis, and Autoimmunity Resulting from Tristetraprolin (TTP) Deficiency, Immunity, 4, 445-454 (1996)
[Non-Patent Document 5] Lian-Qun Qiu et al., Myeloid-specific tristetraprolin deficiency in mice results in extreme lipopolysaccharide sensitivity in an otherwise minimal phenotype, J Immunol. 188(10), 5150-9 (2012)
[Non-Patent Document 6] Nakasa et al., Expression of microRNA-146 in rheumatoid arthritis synovial tissue, Arthritis Rheum. 58(5), 1284-92 (2008)
[Non-Patent Document 7] Mitsumura et al., Ablation of miR-146b in mice causes hematopoietic malignancy, BloodAdv. 2(23), 3483-3491 (2018)
[Non-Patent Document 8] Qing Jing et al., Involvement of MicroRNA in AU-Rich Element-Mediated mRNA Instability, Cell, Vol.120, Page 623-634 (2005)

### [Problem to be Solved by the Invention]

One of the objects of the present invention is to provide an artificial miRNA construct useful for post-transcriptional mRNA regulation.

### [Means for Solving the Problem]

The present inventors have developed novel artificial miRNA constructs useful for post-transcriptional mRNA regulation of genes involved in the onset and progression of chronic inflammatory diseases and the like. The miRNA construct of the present invention is capable of specifically recognizing a target mRNA through a seed sequence that specifically recognizes a binding motif of an RNA-binding protein and regulating the expression thereof. Accordingly, the miRNA construct of the present invention can provide a novel therapeutic or prophylactic approach for chronic inflammatory diseases and the like.

Furthermore, the miRNA construct of the present invention is capable of recognizing multiple target mRNAs to regulate the expression thereof. Therefore, the miRNA construct of the present invention is useful as a novel therapeutic or prophylactic approach for diseases, such as chronic inflammatory diseases, for which it is beneficial to simultaneously suppress the expression of multiple genes.

Moreover, the design method of the miRNA construct of the present invention can be applied to the design of miRNA constructs that target various genes.

Such therapeutic strategies based on the design of artificial miRNAs that are not naturally present in the body have not been previously reported, and were discovered for the first time by the present inventors.

It should be noted that around the time of the publication of Non-Patent Document 8 (i.e., 2005), it was uncovered that the seed sequence of miRNA was bases 2 to 7 or 8 from the 5' end (ugcagca). That is, it was shown that the region of the ARE sequence (bases 11 to 18, uaaauauu) of miR-16 proposed in Non-Patent Document 8 does not function as a seed, and is involved in suppression of mRNA expression (for example, Bcl2) by the sequence ugcagca. Therefore, direct regulation of TNFα by miR-16 as shown in Non-Patent Document 8 has been denied, and no subsequent studies supporting this have been found. In fact, Non-Patent Document 8 itself reports that the action of miR-16 is not observed in the absence of TTP, suggesting that the seed hypothesis is incorrect.

Additionally, development of therapeutics targeting ADAMTS-5 or MMP-13 was discontinued in the course of clinical development for human osteoarthritis (OA), and one reason for this is considered to be the involvement of multiple molecules and factors. In the present invention, it is considered that stronger therapeutic effects can be obtained by simultaneously suppressing many molecules involved in disease exacerbation.

Furthermore, as endogenous miRNAs, miR-146 having anti-inflammatory effects and miR-140 and miR-455 having tissue-protective effects are known; both were first reported by the present inventors as being involved in osteoarthritis, ahead of the rest of the world, and have subsequently been confirmed by many follow-up studies. However, there also exist inflammatory and tissue-destructive factors that are not directly regulated by miR-146, miR-140, or miR-455. On the other hand, the artificial miRNA, miR-ARE, can cover all of IL-1β, MMP-13, and ADAMTS-5, which are involved in the exacerbation of osteoarthritis (OA), and also all of TNFα, IL-6, and MMP-3, which represent pathogenic gene expressions in various cell populations in rheumatoid arthritis (RA). In such cases, the expression of each gene is not reduced to zero but a half level, thereby maintaining a minimal basal immune function, and as a result, side effects such as immunosuppression are expected to be reduced.

The present invention is based on the above findings and encompasses below Aspects.

### [Aspect 1]

A miRNA construct having a sequence, the sequence consisting of:
i) a seed sequence recognizing a binding motif of an RNA-binding protein;
ii) an adenine or uracil residue located on the 5' side of the seed sequence; and
iii) a scramble sequence located on the 3' side of the seed sequence, the scramble sequence having a sequence so that a GC content of the entire miRNA construct is 40-60%.

### [Aspect 2]

The miRNA construct according to Aspect 1, wherein the binding motif of the RNA-binding protein is an AU-rich element, a stem-loop motif, a GU-rich element, or a Pumilio-binding motif.

### [Aspect 3]

The miRNA construct according to Aspect 1, wherein the binding motif of the RNA-binding protein is a binding motif of an RNA-binding protein selected from the group consisting of tristetraprolin (TTP), BRF1, BRF2, AUF1, KSRP, Regnase-1, Roquin, CUGBP1, and Pumilio.

### [Aspect 4]

The miRNA construct according to Aspect 1, wherein the miRNA construct has a length of 19 to 25 nucleotides.

### [Aspect 5]

The miRNA construct according to Aspect 1, wherein the binding motif of the RNA-binding protein is a binding motif present in an mRNA of a gene involved in an inflammatory disease and/or cancer.

### [Aspect 6]

A method for designing a miRNA construct, comprising:
i) the step of selecting a seed sequence recognizing an RNA-binding protein binding motif;
ii) the step of placing an adenine or uracil residue on the 5' side of the seed sequence; and
iii) the step of placing a scramble sequence on the 3' side of the seed sequence, the scramble sequence having a sequence so that a GC content of the entire miRNA construct is 40-60%.

### [Aspect 7]

A method for screening a miRNA library for a miRNA construct, the method comprising:
the step of producing the miRNA library comprising miRNA constructs, the miRNA constructs having a sequence, the sequence consisting of:
   i) a seed sequence recognizing a binding motif of an RNA-binding protein;
   ii) an adenine or uracil residue placed on the 5' side of the seed sequence; and
   iii) a scramble sequence placed on the 3' side of the seed sequence, the scramble sequence having a sequence so that the GC content of the entire miRNA construct is 40-60%; and
the step of selecting a miRNA construct based on whether the miRNA construct has an effect of suppressing expression of a gene involved in at least one type of inflammatory disease, or an effect of improving inflammation in a subject.

### [Aspect 8]

A pharmaceutical composition comprising at least one substance selected from the group consisting of the miRNA construct according to Aspect 1, a mimic of the miRNA construct according to Aspect 1, and DNA encoding the miRNA construct according to Aspect 1.

### [Aspect 9]

The pharmaceutical composition according to Aspect 8, wherein the pharmaceutical composition is an anti-inflammatory agent.

### [Aspect 10]

The pharmaceutical composition according to Aspect 8, further comprising a nucleic acid transfection enhancer.

### [Aspect 11]

The pharmaceutical composition according to Aspect 8, wherein the miRNA construct or the mimic of the miRNA construct comprises a modified nucleic acid.

### [Aspect 12]

The pharmaceutical composition according to Aspect 8, wherein the pharmaceutical composition is used for treatment of a chronic inflammatory disease.

### [Aspect 13]

The pharmaceutical composition according to Aspect 8, wherein the pharmaceutical composition is used for suppressing an expression of a gene involved in at least one type of inflammatory disease.

### [Brief Description of the Drawings]

[FIG. 1] FIG. 1 is a schematic diagram showing the mechanism of mRNA degradation by TTP.
[FIG. 2] FIG. 2 is a diagram showing the sequence of the designed miR-AREs.
[FIG. 3] FIG. 3 is graphs showing the results of suppression of expression induction of inflammatory cytokines and tissue-destructive factors by introduction of miR-AREs.
[FIG. 4] FIG. 4 is a graph showing the results of comprehensive analyses of expression induction-suppressing genes by introduction of miR-ARE2.
[FIG. 5] FIG. 5 shows the results of suppression of arthritis by administration of an miR-ARE: (A) photographs showing the result of Safranin-O staining of the knee joint to which the miRNA was administered; (B) a graph showing the result of scoring the severity of knee arthritis based on OARSI.
[FIG. 6] FIG. 6 is a graph showing the results of comprehensive analysis of expression induction-suppressing genes by introduction of miR-ARE2. "Down": downregulated genes; "non-DEG": genes without expression change; "up": upregulated genes.
[FIG. 7] FIG. 7 is a diagram showing a novel design of seed sequences. #1 to #23 have seed sequences complementary to different AREs, respectively.
[FIG. 8] FIG. 8 is a diagram showing suppression of IL1B and MMP13 expression by a newly designed miR-ARE.
[FIG. 9A] FIG. 9A is a diagram showing the results of comprehensive analyses of genes with suppressed expression by the newly designed miR-AR. Comprehensive analysis of genes with expression changes based on RNA-Seq in cells into which each miR-ARE was introduced. Genes with expression changes were defined as those with log2FC (x-axis) ≥ 1 or ≤ -1 and p-value (y-axis) < 0.05. Genes with expression changes are indicated by •.
[FIG. 9B] FIG. 9B is a diagram showing the results of comprehensive analysis of genes with suppressed expression by the newly designed miR-AR. Specificity analysis of miR-ARE; the darker the color, the stronger the expression suppression.
[FIG. 9C] FIG. 9C is a diagram showing the results of comprehensive analysis of genes with suppressed expression by the newly designed miR-AR. GO analysis of genes with decreased expression upon introduction of miR-ARE-N2, N8, and N21.
[FIG. 9D] FIG. 9D is a diagram showing the results of comprehensive analysis of genes with suppressed expression by the newly designed miR-AR. Gene expression changes and downregulated genes upon introduction of miR-ARE-N2, N8, and N2
[FIG. 10] FIG. 10 is graphs showing suppression of target genes by LNP-formulated miRNA.

### [Modes for Carrying Out the Invention]

One aspect of the present invention relates to a novel artificial miRNA constructs

MicroRNAs (miRNAs) present in vivo are short non-coding RNAs of 20 to 24 nucleotides, and they are involved in post-transcriptional regulation of gene expression in multicellular organisms by affecting both mRNA stability and translation. miRNAs are transcribed by RNA polymerase II as part of a capped and polyadenylated primary transcripts (pre-miRNA). The transcripts may or may not code for proteins. The primary transcripts are cleaved by the Drosha RNase III enzyme to generate precursor miRNAs (pre-miRNA) of approximately 70 nucleotides with a stem-loop structure. They are further cleaved by the cytoplasmic Dicer RNase to yield mature miRNAs and antisense counterparts thereof, miRNAs*. The mature miRNAs are incorporated into the RNA-induced silencing complexes (RISCs), which recognizes target mRNAs through imperfect base pairing with the miRNAs, generally resulting in translational repression or destabilization of the target mRNAs.

The present inventors have developed novel artificial miRNA constructs capable of post-transcriptional mRNA regulation of target genes, like the above-mentioned mature (endogenous) miRNAs. The miRNA constructs of the present invention are characterized in that they have a sequence consisting of:
i) a seed sequence that recognizes a binding motif of an RNA-binding protein,
ii) an adenine or uracil residue positioned on the 5' side of the seed sequence, and
iii) a scramble sequence positioned on the 3' side of the seed sequence.

### i) Seed Sequence

The miRNA constructs of the present invention comprise a seed sequence that recognizes a binding motif of an RNA-binding protein that binds to the target mRNA.

### RNA-binding Proteins and Their Binding Motifs

As described above, RNA-binding proteins are known to recognize and act upon specific sequences (binding motifs) present in the 3' untranslated region (3'UTR) of target mRNAs, thereby destabilizing the mRNAs and suppressing the expression of the target genes.

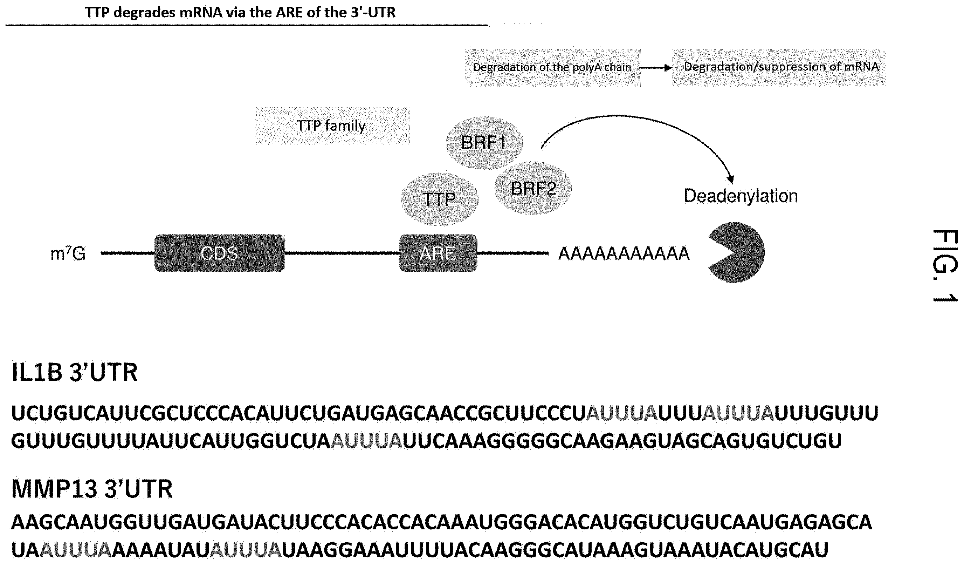

For one example, FIG. 1 shows a schematic diagram illustrating the mechanism of mRNA degradation by the RNA-binding protein TTP. As shown in FIG. 1, the TTP binds to the AU-rich element (ARE: AU Rich Element) (AUUUA) present in the 3'UTR of the target mRNA of an inflammatory cytokine such as IL-1β or a tissue-destructive factor such as MMP13 (the 3'UTR sequence of IL-1β is shown as Sequence ID No: 1, and the 3'UTR sequence of MMP13 is shown as Sequence ID No: 2), and recruits the deadenylase enzyme to the target mRNA, thereby inducing degradation of the poly-A tail and promoting degradation of the mRNA.

The miRNA constructs of the present invention include a seed sequence that recognizes such binding motifs of RNA-binding proteins, thereby specifically recognizing target mRNAs possessing such binding motifs and controlling their expression.

The binding motifs of RNA-binding proteins that can serve as targets for the miRNA constructs of the present invention may be any binding motifs present in the mRNA of the target gene. In one embodiment, the binding motif may be that of an RNA-binding protein involved in post-transcriptional mRNA regulation. In another embodiment, the binding motif is one that exists in the 3' untranslated region of the mRNA of target gene.

Examples of such binding motifs include AU-rich elements located in the 3' untranslated regions of inflammatory cytokines or matrix-degrading enzymes involved in chronic inflammation, to which the aforementioned TTP and its family proteins BRF1 (Zfp36L1), BRF2 (Zfp36L2), as well as AUF1 and KSRP, can bind; stem-loop motifs in the 3' untranslated regions of IL-6, ICOS, TNF-α, etc., to which Regnase-1 and Roquin can bind; GU-rich elements (GRE: GU Rich Element) (e.g., UGUUUGUUUGU [Sequence ID No: 3]) to which CUGBP1 can bind; and Pumilio binding motifs (e.g., PRE: Pumilio response element, UGUANAUA) to which Pumilio can bind.

In one embodiment, the RNA-binding protein binding motif is an AU-rich element.

### Seed Sequence

The seed sequence is located on the 5' side of the miRNA and is responsible for target recognition of the miRNA. The miRNA constructs of the present invention include a seed sequence that recognizes the above-described binding motif of the RNA-binding protein, and thereby specifically recognizes the target mRNA having the binding motif. That is, the seed sequence forms a complementary strand with the target mRNA having the binding motif and is an important region determining target specificity.

The seed sequences of the miRNA constructs of the present invention comprise a sequence that is at least partially complementary to the binding motif of the RNA-binding protein. The length of the complementary sequence is preferably 4 or more bases, more preferably 5 or more bases, and even more preferably 6 or more bases. Additionally, the seed sequence is preferably at least 60% complementary to the consensus sequence of the binding motif of the intended RNA-binding protein, more preferably at least 80% complementary, and in some cases, even more preferably 100% complementary (i.e., the entire length of the seed sequence is fully complementary to all or a part of the consensus sequence of the binding motif).

The case in which the binding motif of the RNA-binding protein is an AU-rich element is described as an example. It is known that the AU-rich element have a consensus sequence containing AUUUA. The consensus sequence may include a sequence such as WAUUUAWW. In one embodiment, the consensus sequence consists of 9 bases: WWAUUUAWW. Here, W is either A or U, either of which is allowed. The third and seventh bases (A) may be substituted with U, provided that at least one of them remains A.

As the seed sequence that recognizes this AU-rich element, a sequence that is at least partially complementary to the consensus sequence of the AU-rich element (NWAUUUAWW) can be used. In one embodiment, the seed sequence is preferably 5 to 9 bases in length, more preferably 6 to 8 bases, and for example, 7 bases in length. In one embodiment, the seed sequence preferably includes at least a sequence complementary to the third to seventh bases (AUUUA) of the above-mentioned consensus sequence of the AU-rich element (with allowance for substitution of A with U at one of the third and seventh positions).

Examples of such seed sequences include, for instance, UAAAUAA in miR-ARE-1, UAAAUAU in miR-ARE-2, and AAUAAAU in miR-ARE-3, which are described later, but are not limited thereto.

Target genes whose protein expression can be regulated by being recognized by the seed sequences of the miRNA constructs of the present invention, include genes involved in inflammatory diseases, such as inflammatory cytokines (e.g., IL-6, TNFα, IL-1β (also described as IL-1B), IL-12A, IL-18, IL-11, IL23A, LTA); chemokines (e.g., CXCL8, CXCL5, CCL20, CCL2, CCL1, CCL7); tissue-destructive factors such as matrix-degrading enzymes (e.g., MMP3, MMP13, ADAMTS4, ADAMTS5); hematopoietic and growth factors (e.g., CSF2, CSF3, HBEGF, AREG); receptors, signaling molecules, and transcription factors (e.g., IL1RAP, IL1R, ITGB8, TRAF4, CD83, NFKB2); cytokine receptors (e.g., IL6R, IL6ST, TNFRSF1B); and acute-phase proteins (e.g., SAA1, SAA2, S100A8, S100A9). The miRNA constructs of the present invention can simultaneously suppress the expression of one or more mRNAs of these genes selected from the above gene groups.

In diseases such as chronic inflammation, therapeutic strategies that simultaneously suppress the expression of multiple genes of inflammatory cytokines and tissue-destructive factors, are effective. Unlike siRNAs, which recognize perfectly matched sequences and destroy their targets, the miRNA constructs of the present invention has a broader mechanism of recognizing seed sequences, and thus one miRNA can recognize a series of target mRNAs and suppress the expression of multiple proteins. Therefore, the miRNA constructs of the present invention can provide a novel therapeutic and preventive approach useful for diseases that require regulation of expression of multiple proteins, such as chronic inflammation.

Moreover, the miRNAs of the present invention can also target genes other than the above-mentioned target genes, as long as the mRNAs contain the desired binding motif. For example, since AREs are present in mRNAs such as MYC, Cyclin D, and EGF, targeting AREs with the miRNA of the present invention allows regulation of the expression of multiple genes associated with inflammatory diseases and cancers.

### ii) Adenine or Uracil Base on the 5' Side of the Seed Sequence

The miRNA constructs of the present invention include an adenine or uracil base on the 5' side of the seed sequence. In one embodiment, it is preferable to have an adenine base on the 5' side of the seed sequence. In another embodiment, it is more preferable to have an uracil base on the 5' side of the seed sequence. Including an adenine or uracil base on the 5' side of the seed sequence may enhance the function as a miRNA. Although the reason is not clear, it is considered that a 5' end consisting of an adenine or uracil base improves miRNA strand separation, RISC complex formation, and mRNA recognition.

### iii) Scramble Sequence on the 3' Side of the Seed Sequence

The miRNA constructs of the present invention include a scramble sequence on the 3' side of the seed sequence. The scramble sequence may be any random sequence; however, it is preferably constructed so that the overall GC content of the miRNA construct is 40% to 60%, preferably 45% to 55%, more preferably 48% to 52%, and particularly preferably about 50%. The length of the scramble sequence may be determined so that the total nucleotide length of the miRNA construct is 19 to 25 bases, preferably 20 to 24 bases, for example, 21 to 23 bases.

By configuring the scramble sequence in this way, the incorporation efficiency into RISC and the efficiency of translation inhibition or destabilization of the target mRNA can be improved.

Furthermore, in one embodiment, it is preferable that the scramble sequence is designed so as not to form complementary base pairs with transcripts other than the target mRNA.

Non-limiting examples of the artificial miRNA constructs of the present invention include:
miR-ARE1: 5'-AUAAAUAAgccuccgggaucca-3' (Sequence ID No: 4) (seed target sequence: 5'-UUAUUUA-3'),
miR-ARE2: 5'-AUAAAUAUgccuccgggaucca-3' (Sequence ID No: 5) (seed target sequence: 5'-AUAUUUA-3'),
miR-ARE3: 5'-AAAUAAAUgccuccgggaucca-3' (Sequence ID No: 6) (seed target sequence: 5'-AUUUAUU-3').
In addition, sequences in which the adenine base on the 5' side of the seed sequence in the above sequences is replaced with a uracil base can also be preferably used.

Additional non-limiting examples of the artificial miRNA constructs of the present invention include:
miR-ARE4: 5'-uAAAAUUAgccuccgggauccaTT-3' (Sequence ID No: 8) (seed target sequence: 5'-UUUUAAU-3'),
miR-ARE5: 5'-uAUAAAUUgccuccgggauccaTT-3' (Sequence ID No: 9) (seed target sequence: 5'-AAUUUAU-3').
In the above sequences, the first nucleotide 'u' is phosphorylated at the 5' end to facilitate incorporation into the miRNA RISC.

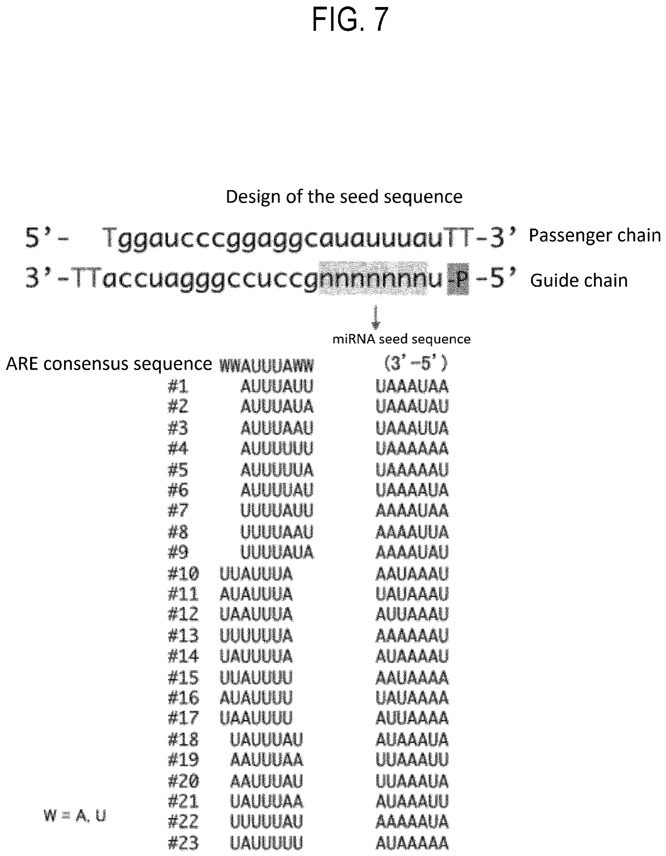

Further non-limiting examples of the artificial miRNA constructs of the present invention include those comprising seed sequences listed in FIG. 7 (in the 3'-5' orientation, UAAAUAA, UAAAUAU, UAAAUUA, UAAAAAA, UAAAAAU, UAAAAUA, AAAAUAA, AAAAUUA, AAAAUAU, AAUAAAU, UAUAAAU, AUUAAAU, AAAAAAU, AUAAAAU, AAUAAAA, UAUAAAA, AUUAAAA, AUAAAUA, UUAAAUU, UUAAAUA, AUAAAUU, AAAAAUA, AUAAAAA).

In another aspect, the present invention relates to a method for designing an artificial miRNA construct. As described above, the miRNA construct comprises:
i) a seed sequence that recognizes a binding motif of an RNA-binding protein,
ii) an adenine or uracil residue located on the 5' side of the seed sequence, and
iii) a scramble sequence located on the 3' side of the seed sequence,
and by designing the scramble sequence such that the overall GC content of the miRNA construct is between 40% and 60%, it become possible to obtain a miRNA construct that recognizes a target mRNA having the desired RNA-binding protein binding motif and regulates its expression. The design method of the artificial miRNA construct of the present invention is not limited to target mRNAs involved in the above-mentioned inflammatory diseases, but may be applied to design miRNA constructs targeting various mRNAs.

In another aspect, the present invention also relates to a method for screening a library of miRNA constructs for the artificial miRNA construct of the present invention.

In one embodiment, the screening method of the present invention comprises the step of producing a library of miRNA constructs, the miRNA constructs consisting of:
i) a seed sequence recognizing a binding motif of a certain RNA-binding protein,
ii) an adenine or uracil residue located on the 5' side of the seed sequence, and
iii) a scramble sequence located on the 3' side of the seed sequence, the scramble sequence having a sequence so that the total GC content of the miRNA construct is 40% to 60%,
and the step of selecting a miRNA construct, based on whether the miRNA construct has an effect of suppressing the expression of at least one gene involved in an inflammatory disease or an effect of improving inflammation in a subject.

In one embodiment, the step of determining whether the miRNA construct has an effect of suppressing the expression of at least one gene involved in an inflammatory disease or an effect of improving inflammation in a subject comprises contacting the library of miRNA constructs with a sample containing a target mRNA. As used herein, "target mRNA" refers to an mRNA whose expression is intended to be regulated by the miRNA construct of the present invention. The sample containing the target mRNA may be an in vitro sample, an ex vivo sample, or an in vivo sample.

In one embodiment, the effect of suppressing the expression of a gene involved in an inflammatory disease may be evaluated using quantitative real-time PCR, immunostaining, or any other method known to those skilled in the art.

In one embodiment, the effect of improving inflammation in a subject may be evaluated using the OARSI score in an arthritis model or any other method known to those skilled in the art.

In another aspect, the present invention also relates to a method for screening candidate genes for a gene under the influence of a binding motif of a given RNA-binding protein.

In one embodiment, the screening method of the present invention comprises the step of preparing a miRNA construct, the miRNA construct consisting of:
i) a seed sequence recognizing a binding motif of a certain RNA-binding protein,
ii) an adenine or uracil residue located on the 5' side of the seed sequence, and
iii) a scramble sequence located on the 3' side of the seed sequence, the scramble sequence having a sequence so that the total GC content of the miRNA construct is 40% to 60%,
   the step of contacting the miRNA construct with a sample containing mRNA of candidate genes, and
   the step of determining that a candidate gene is under the influence of the given RNA-binding protein binding motif when the expression of the candidate gene is decreased upon contact with the miRNA construct

In the present embodiment, the term "gene under the influence of a given RNA-binding protein binding motif" refers to a gene whose expression can be regulated by any action (e.g., binding of a certain molecule such as an RNA-binding protein or miRNA) to the given binding motif.

Expression of the candidate genes may be evaluated using quantitative real-time PCR, immunostaining, or any other method known to those skilled in the art.

In another aspect, the present invention relates to a pharmaceutical composition comprising the artificial miRNA construct of the present invention.

As described later, the present inventors have demonstrated that the expression of target mRNA involved in chronic inflammatory diseases is suppressed by introducing the artificial miRNA construct of the present invention into cells in the absence of the corresponding RNA-binding protein. That is, by supplementing the absence of the corresponding RNA-binding protein with the artificial miRNA construct of the present invention, it is possible to provide a novel therapeutic or prophylactic approach against chronic inflammatory diseases. Thus, several embodiments of the present invention relate to a pharmaceutical composition comprising the artificial miRNA construct of the present invention.

In the case of endogenous miRNAs, a double-stranded RNA intermediate with a length of approximately 22 nucleotides is formed during the maturation from pre-miRNA to mature miRNA, and one strand (corresponding to the mature miRNA) is incorporated into a complex called RISC and functions as a miRNA, while the other strand is degraded. In the case of the artificial miRNA constructs of the present invention, the RNA synthesized based on the sequence of the present miRNA construct may be used directly as a mature miRNA. When synthesizing RNA, in addition to natural nucleic acids, modified nucleic acids such as 2'O-methyl-modified nucleotides or bridged nucleic acids (BNA) may also be used. Methods for synthesizing such nucleic acids are well known to those skilled in the art.

The pharmaceutical composition of the present invention may comprise a mimic of the artificial miRNA construct of the present invention instead of the construct itself. As used herein, a "mimic" refers to a short synthetic double-stranded RNA that mimics the artificial miRNA construct of the present invention. In one embodiment, the miRNA construct mimic of the present invention has at least 80% homology, more preferably at least 90%, and still more preferably at least 95% (including 100%) homology with the mimicked miRNA construct.

The artificial miRNA constructs and its mimics of the present invention may include, as modified nucleotides, 2'-MOE, 2'-O-MCE, LNA (2'-4' BNA), ENA, AmNA, GuNA, scpBNA, 2'-OMe, phosphorothioate modifications, and the like.

Further, in place of the artificial miRNA construct of the present invention, the pharmaceutical composition of the present invention may include a DNA encoding the miRNA construct. For example, the sequence of the miRNA construct of the present invention may be introduced into various expression vectors or various viral vectors, and RNA may be synthesized intracellularly via transcription.

The artificial miRNA constructs, the mimics of the miRNA construct, and the DNAs encoding the miRNA constructs according to the present invention may be chemically, biochemically, or biologically synthesized. Such synthesis methods are widely known to those skilled in the art.

When administering the miRNA construct, the mimic of the miRNA construct, or the DNA encoding the miRNA construct according to the present invention to a patient, it is preferable to include in the pharmaceutical composition a substance that enhances the nucleic acid introduction efficiency of miRNAs or the like. Preferred examples of nucleic acid transduction enhancers include, for example, Atelocollagen, Invivofectamine, in vivo JetPEI, GeneJet, and Avalanche-in vivo Transfection Reagent. Furthermore, the miRNA may be encapsulated in liposomes or nanoparticles. In addition, the miRNA may be directly or indirectly conjugated with a targeting ligand such as N-acetylgalactosamine (GalNAc) and cholesterol.

The pharmaceutical composition according to the present invention can take any form, such as tablets, powders, liquids, or semi-solids, and may include suitable excipients and additives in addition to the artificial miRNA construct, the mimic of the miRNA construct, and/or the DNA encoding the miRNA construct. The pharmaceutical composition of the present invention may also contain other anti-inflammatory agents. The amount of each component may be appropriately determined within pharmaceutically acceptable ranges. The dosage of the composition may also be appropriately determined depending on the type of nucleic acid used and the subject to be administered. The administration route may also be appropriately determined depending on the type of nucleic acid and the subject.

### Target Diseases

Expression regulation of a desired target mRNA can be performed by changing the target of the seed sequence in the artificial miRNA constructs of the present invention. Therefore, the pharmaceutical compositions according to the present invention can be used for the treatment and prevention of various diseases, and in particular, it can be effectively used for the treatment and prevention of diseases involving cytokines or matrix-degrading enzymes. Examples of diseases involving cytokines or matrix-degrading enzymes include chronic inflammatory diseases such as age-related diseases (e.g., osteoarthritis), autoimmune diseases (e.g., rheumatoid arthritis), respiratory diseases (e.g., asthma), allergic diseases (e.g., atopic dermatitis), metabolic syndromes (e.g., diabetes), viral infections, and cytokine storms in sepsis. Therefore, the pharmaceutical compositions according to the present invention may serve as an anti-inflammatory agent. Additionally, the pharmaceutical compositions according to the present invention can be suitably used for the treatment and prevention of diseases associated with loss or reduced expression of RNA-binding proteins, including cancers (breast, cervical, colorectal, gastric, hepatic, pulmonary, pancreatic, skin cancers such as DMBA/TPA-induced skin cancer), cachexia, keratosis, gastritis, elevated anti-DNA antibodies, Crohn's disease, colitis (e.g., DSS-induced colitis), fatty liver, and the like.

The pharmaceutical compositions according to the present invention are particularly useful for diseases such as chronic inflammatory diseases, which require the regulation of expression of multiple genes.

In the present specification, the above-mentioned diseases may be collectively referred to as "chronic inflammatory diseases and the like."

One aspect of the present invention relates to a pharmaceutical composition for use in the treatment and/or prevention of chronic inflammatory diseases and the like comprising, as an active ingredient, the artificial miRNA construct, a mimic of the miRNA construct, and/or a DNA encoding the miRNA construct according to the present invention. That is, one aspect of the present invention relates to use of the artificial miRNA construct, its mimic, and/or a DNA encoding the same of the present invention in manufacturing a therapeutic and/or prophylactic agent for chronic inflammatory diseases and the like.

The content of the active ingredient (i.e., the artificial miRNA construct, the mimic of the miRNA construct, and/or the DNA encoding the miRNA construct according to the present invention) in the pharmaceutical composition may be appropriately determined within the range of 0.001 to 99.99 wt%. The pharmaceutical composition may include agents that facilitate the introduction of nucleic acids or proteins into cells. There is no particular limitation on other components of the pharmaceutical composition of the present invention; they can be appropriately selected depending on the purpose and may include, for example, pharmaceutically acceptable carriers or additives. The carriers or additives may be appropriately selected depending on the dosage form and may include any carriers, diluents, excipients, suspending agents, lubricants, adjuvants, vehicles, delivery systems, nucleic acid introduction enhancers, emulsifiers, disintegrants, absorption enhancers, preservatives, surfactants, colorants, fragrances, or sweeteners., but are not limited thereto Their content in the pharmaceutical composition of the present invention is not particularly limited and may be appropriately selected depending on the purpose.

The dosage form of the pharmaceutical composition according to the present invention is not particularly limited and may be appropriately selected depending on the desired route of administration. Examples include injections (solutions, suspensions, solids for dissolution upon use, etc.), and solid preparations (tablets, capsules, suppositories, powders, etc.). For injectable preparations, for example, pH adjusters, buffers, stabilizers, isotonic agents, local anesthetics, etc., may be added to the composition to produce injections for subcutaneous, intra-articular, intramuscular, intravenous, or other uses by conventional methods. Examples of pH adjusters and buffers include sodium citrate, sodium acetate, and sodium phosphate. Examples of stabilizers include sodium metabisulfite, EDTA, thioglycolic acid, and thiolactic acid. Examples of isotonic agents include sodium chloride and glucose. Examples of local anesthetics include procaine hydrochloride and lidocaine hydrochloride. Solid preparations may be enteric-coated.

There is no particular limitation on the administration method of the pharmaceutical composition according to the present invention. For example, local or systemic administration may be selected depending on the dosage form and the condition of the patient. Administration may be carried out, for example, by intra-articular administration, intravenous administration, subcutaneous administration, intramuscular administration, oral administration, enteral administration, enema, or tube feeding.

There is no particular limitation on the subject of administration of the pharmaceutical composition of the present invention. It may be appropriately selected depending on the purpose, and may include humans, as well as non-human mammals such as mice, rats, bovine, pigs, monkeys, dogs, and cats, but humans being is preferred, and particularly human patients with joint disease are preferred. Furthermore, the pharmaceutical composition of the present invention may be administered for the purpose of preventing the onset of the target disease, particularly for the purpose of preventing recurrence.

The dosage of the pharmaceutical composition according to the present invention is not particularly limited and may be appropriately selected depending on the form of administration, age, body weight, and the desired degree of effect in the subject to be administered. The dosage of the pharmaceutical composition according to the present invention may be, for example, from 0.01 to 1,000,000 nmol per day, preferably from 0.1 to 100,000 nmol per day, more preferably from 1 to 50,000 nmol per day, and particularly preferably from 10 to 10,000 nmol per day. The frequency of administration may be, for example, 1 to 100 times per month, preferably 1 to 50 times per month, and the administration period may range from 1 day to 24 months, preferably from 1 day to 12 months, for example, from 1 day to 6 months.

There is no particular limitation on the timing of administration of the pharmaceutical composition of the present invention, and it may be appropriately selected according to the intended purpose. For example, it may be prophylactically administered to patients who are susceptible to the above diseases or therapeutically administered to patients presenting symptoms. There is also no particular limitation on the number of administrations, which may be appropriately selected depending on the age, body weight, and desired degree of effect for the subject to be administered.

### Methods for Treatment and Prevention

One aspect of the present invention relates to a method for treating and/or preventing chronic inflammation or the like in a subject requiring treatment and/or prevention, respectively, the method comprising administering to the subject an effective amount of an active ingredient (i.e., the artificial miRNA construct, a mimic thereof, and/or DNA encoding the miRNA construct according to the present invention). The subject in need of treatment and/or prevention is a mammal, such as a human. The dosage may be appropriately determined depending on the type of drug used and the subject. The route of administration may also be appropriately determined depending on the type of drug used and the subject. Preferred routes of administration include, for example, intra-articular, subcutaneous, or intravenous administration.

Hereinafter, the present invention will be described in more detail by way of examples. However, the present invention is not limited to these examples.

### Examples

### Example 1: Design and Synthesis of miR-ARE

As noted above, AU-rich elements (AREs) are known to have a 9-base consensus sequence of WWAUUUAWW. W is either A or U, and both of A and U are allowed . Either the third or the seventh A can be substituted with U, as long as one remains A.

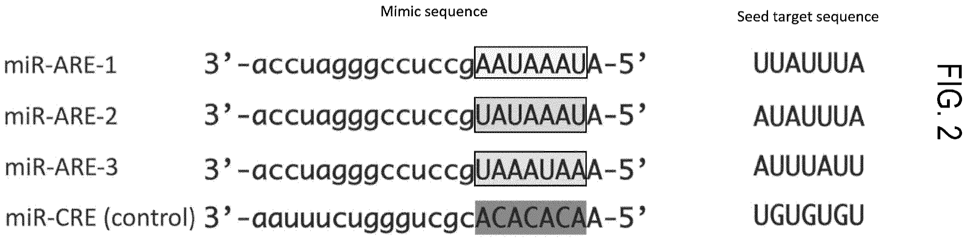

Based on these features, miR-ARE1 to 3 (SEQ ID NOs: 4, 5, 6) were designed to target AREs. The 2nd to 8th bases from the 5' end of the miRNA are referred to as the seed sequence, which forms complementary pairing with the target mRNA and is important for target specificity. Therefore, miRNAs with seed sequences complementary to AREs were designed. The region 3' to the seed was designed as a scramble sequence that does not form complementary base pairing with transcripts in humans or mice so that overall GC content of the miRNA is approximately 50%. The sequences of each miR-ARE are shown in Figure 2.

As a negative control, miR-CRE with a GU repeat sequence (5'-AACACACAcgcugggucuuuaa-3': SEQ ID NO: 7) was designed. The miRNAs were synthesized as miRNA mimics by Ambion.

### Example 2: Suppression of Expression of Inflammatory Cytokines and Tissue-Damaging Factors by miR-ARE Introduction (qPCR)

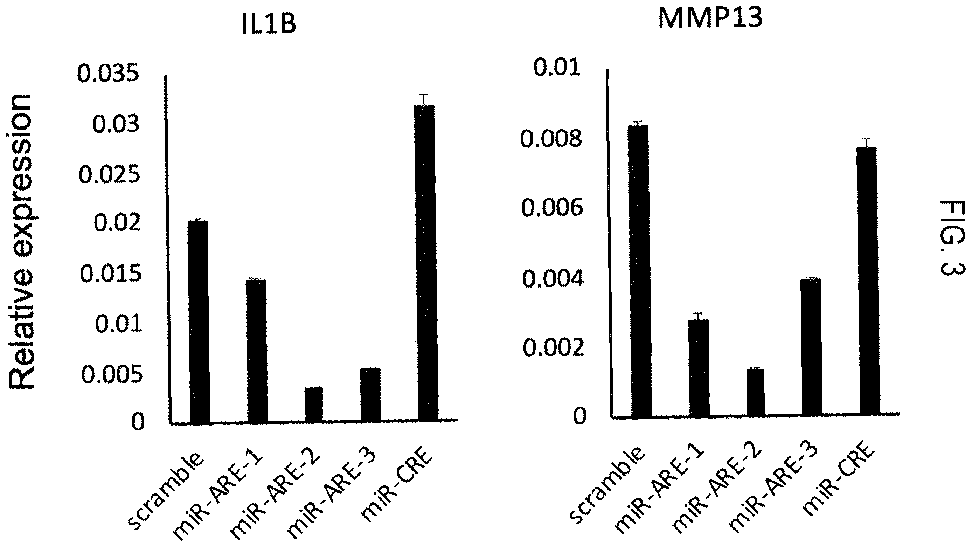

Human chondrosarcoma SW1353 cells were transfected with miR-ARE1 to 3 (final concentration 50 nM) and, as negative controls, miR-CRE and scramble RNAs (scramble sequences of miR-ARE1 to 3), using Lipofectamine RNAiMAX (Thermo Fisher Scientific), and cultured for 48 hours. Cells were then stimulated for 6 hours with 20 ng/mL human IL-1β, RNA was purified, and expression of IL-1B and MMP13 was analyzed by quantitative PCR (qPCR) after reverse transcription. Expression levels were calculated as relative values normalized to GAPDH. Results are shown in Figure 3.
Expression of IL-1B and MMP13 decreased upon introduction of miR-ARE1 to 3, whereas the negative controls (miR-CRE and scramble) did not show such decreases.

### Example 3: Comprehensive Analysis of Gene Expression Suppression by miR-ARE2 (RNA-Seq)

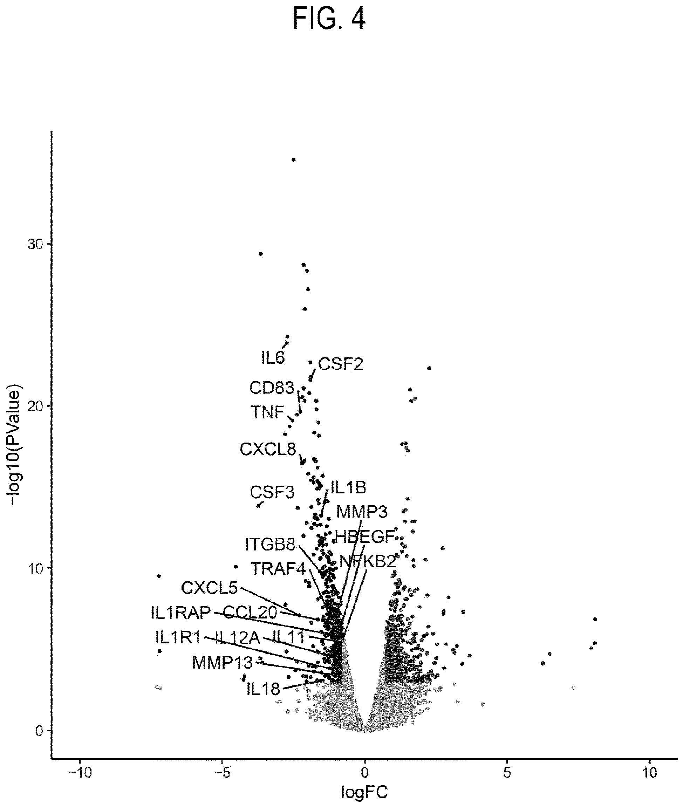

The miR-ARE2 whose suppression efficiency had been the highest was introduced into SW1353 cells in the same way as described above, and comprehensive analyses of target genes was conducted by RNA-Seq. For RNA-Seq , FASTQ files obtained from next-generation sequencer were mapped to the human genome (hg38) using STAR (v2.7.6). Gene expression was quantified with RSEM, and genes with expression changes were analyzed using edgeR. Results are shown in Figure 4. Genes with logFC < -0.5 were considered as significantly downregulated, and logFC > 0.5 as significantly upregulated (FDR < 0.05).

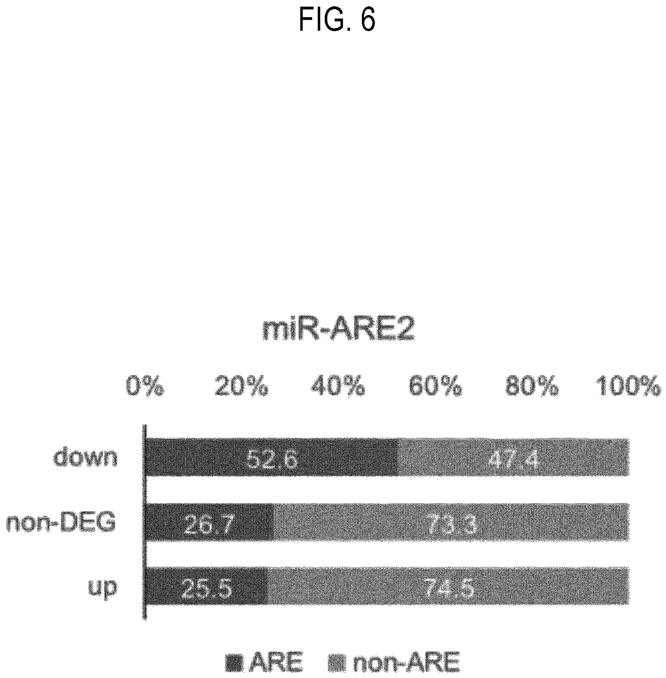

Introduction of miR-ARE2 downregulated expression of inflammatory cytokines (IL-6, TNFα, IL-1β, IL-12A, IL-18, IL-11), chemokines (CXCL8, CXCL5, CCL20), tissue-damaging factors (MMP3, MMP13), hematopoietic/growth factors (CSF2, CSF3, HBEGF), and signal transduction factors (IL1RAP, IL1R, ITGB8, TRAF4, CD83, NFKB2). In upregulated genes (up), downregulated genes (down) and genes with no expression change (non-DEG), genes containing the ARE that is a target sequence of miR-ARE were selected and over 50% of the downregulated genes contained AREs (Figure 6).

### Example 4: Evaluation of Arthritis Suppression by miR-ARE Administration (DMM Model)

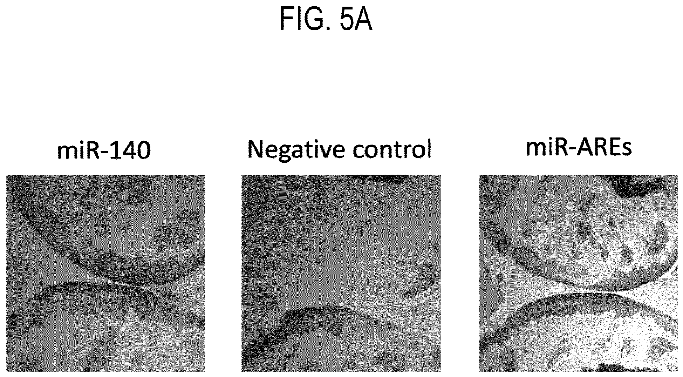

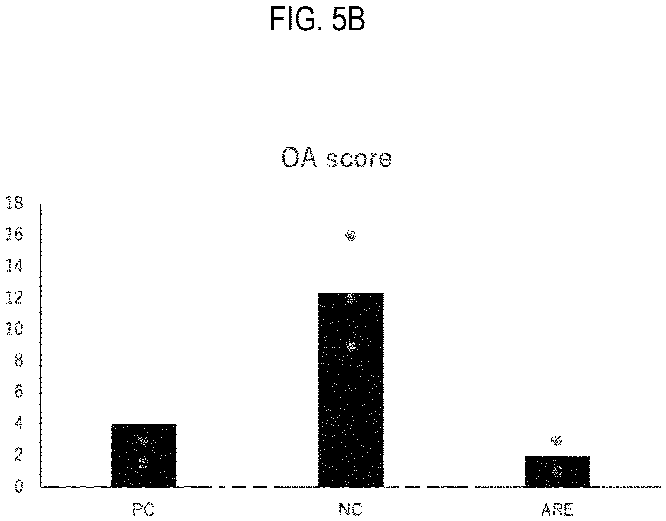

To evaluate therapeutic effects of miR-ARE on arthritis, a destabilization of the medial meniscus (DMM) model was used for the evaluation. A 10-week-old C57BL/6 J mouse underwent surgical induction of arthritis by destabilization of the medial meniscus (DMM) in the right knee. A mixture containing equimolar amounts of 50 µM miR-ARE-1, miR-ARE-2, and miR-ARE-3 (collectively "miR-AREs"; final administration volume 1-10 µL), 50 µM negative control miRNA (Thermo Fisher Scientific; mirVana^{™} miRNA mimic Negative Control #1), or positive control miRNA mimic miR-140 (Ambion; miRNA mimic), were each mixed with an equivalent volume of Complexation Buffer and twice the volume of Invivofectamine 3.0 reagent (both Thermo Fisher Scientific) and incubated at 50 °C for 30 minutes. Thereafter, 15 µL PBS, 12.5 µL Atelo Gene QG (KOKEN), and 12.5 µL dilution buffer were added. These preparations were administered into the joint cavity of mice seven days after DMM surgery. The miRNA administrations were performed twice per week for eight weeks. At postoperative week 8, joints were harvested, fixed in 4% paraformaldehyde, decalcified with formic acid/sodium citrate solution, and sectioned. The sections were stained with Safranin-O or hematoxylin and eosin (see results in Figure 5(A)). In the negative control group, loss of extracellular matrix in the cartilage surface and progression of arthritis were observed; in contrast, mice treated with miR-AREs retained cartilage tissue, similarly to the positive control miR-140 group. Arthritis severity was scored based on the OARSI evaluation criteria. Mice treated with miR-AREs showed lower scores than the negative control group, indicating suppression of severity (see results in Figure 5(B)).

### Example 5: Novel Design of miR-ARE

Twenty-three types of 7-base seed sequences targeting the ARE consensus sequence (WWAUUUAWW) were designed under the following rules:
1. Seed sequence: 7 bases
2. W in the ARE consensus sequence is A or U
3. Either the 2nd or 7th A may be U, but at least one must be A
4. Non-seed regions: random sequences not complementary to human/mouse t ranscripts
5. GC content of the entire miRNA: 40-60%

In accordance with these rules, seed sequences for 23 designable different miRNAs were designed. The first nucleotide of the guide strand is either adenine or uracil (in this instance, uracil was selected).

The uracil at the 1st position is phosphorylated at the 5' end to facilitate incorporation into the miRNA-RISC. The 5' end of the passenger strand is modified with LNA to prevent its incorporation into the miRNA-RISC.

Additionally, two LNA-modified T are introduced at the 3' ends of both the guide and passenger strands to prevent degradation by exonucleases.

See Fig.7 for sequences. Fig.7 shows an exemplary passenger strand (5'-TggaucccggaggcauauuuauTT-3'; SEQ ID NO: 10) and guide strand (5'-unnnnnnngccuccgggautccaTT-3'; SEQ ID NO: 11). Capital T indicates LNA; lowercase n = any base.

### Example 6: Suppression of Gene Expression by Novel miRNAs

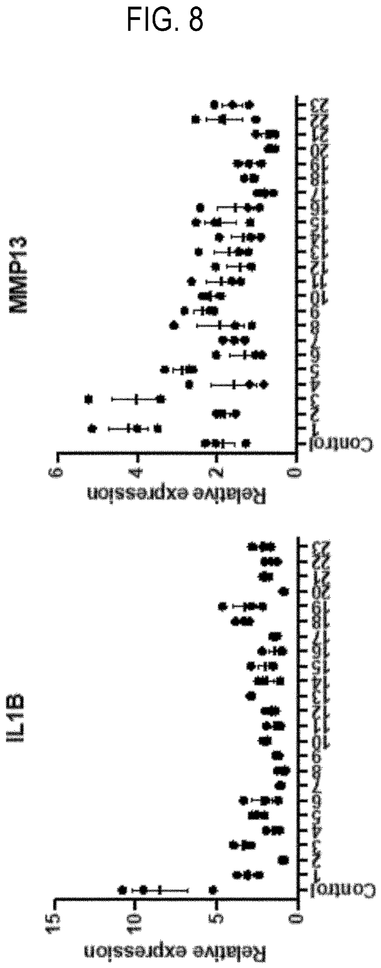

Each miRNA was introduced into SW1353 cells using Lipofectamine RNAiMAX at 10 nM. After 48 h, cells were stimulated with 20 ng/mL human IL-1β for 6 h. RNA was extracted, reverse transcribed, and IL1B and MMP13 quantified by qPCR with HPRT as control.
As shown in Figure 8, all miRNAs suppressed IL-1B expression; MMP13 suppression was observed in some.
Highly effective seed sequences include #17 (UAAUUUU) and #20 (AAUUUAU).

### Example 7: RNA-Seq

SW1353 cells were transfected with each miRNA at a final concentration of 10 nM using Lipofectamine RNAi Max (Thermo Fisher Scientific). Each transfection was performed in triplicate (N = 3). After 48 hours, cells were stimulated with 20 ng/mL human IL-1β (Wako Pure Chemical Industries) for 6 hours. Total RNA was isolated, and then an RNA-Seq library was prepared using the QuantSeq 3' mRNA-Seq Library Prep Kit (FWD) (Lexogen).

Next-generation sequencing was performed on a NextSeq 1000 system in single-end 100 nt mode. Subsequent demultiplexing was conducted based on sample-specific index sequences.

The resulting FASTQ files were mapped to the human genome reference hg38.

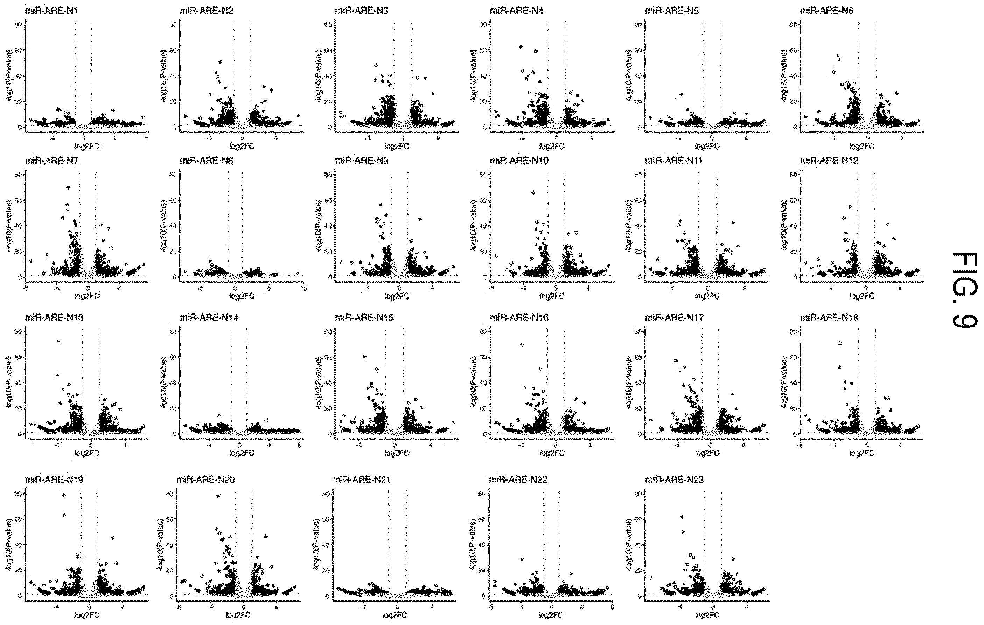

Count data were obtained for each gene from the mapping results using HTSeq, and genes with expression changes were quantified using edgeR (see Fig. 9A).

Sequences comprising the seed-sequence of each miR-ARE were segmented into contiguous 7-nt windows. Genes harboring sequences that are complementary to the seven bases in their 3'-UTRs were identified from genes showing reduced expression. Analysis of expression reduction of the identified genes revealed that among the 23 miRNAs tested, miR-ARE-N2, N8, and N21 exhibited particularly high expression suppression efficiency and specificity (see Fig. 9B). For these three miRNAs, Gene Ontology analysis of downregulated genes was performed using Metascape (https://metascape.org/gp/index.html#/main/step1), revealing strong enrichment for genes involved in inflammation and extracellular matrix remodeling (see Fig. 9C). Furthermore, by introduction of miR-ARE-N2, N8, and N21, it was revealed that inflammatory cytokines such as IL-6 and CXCL8 (IL-8) and chemokines, as well as matrix-degrading factors including MMPs and ADAMTS family members are included (see Fig. 9D).

### Example 8: Suppression of Expression of Target Gene by Introduction of LNP-formulated miRNA

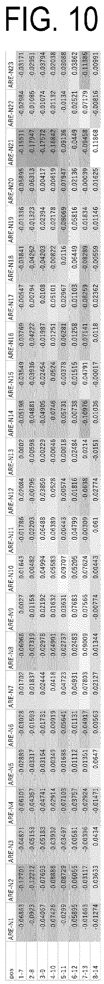

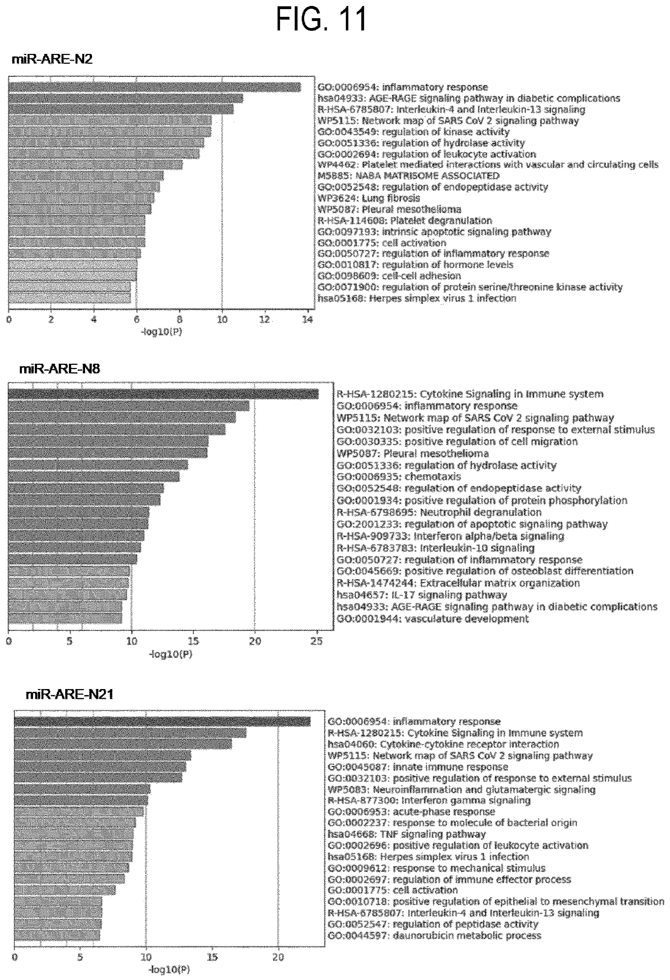

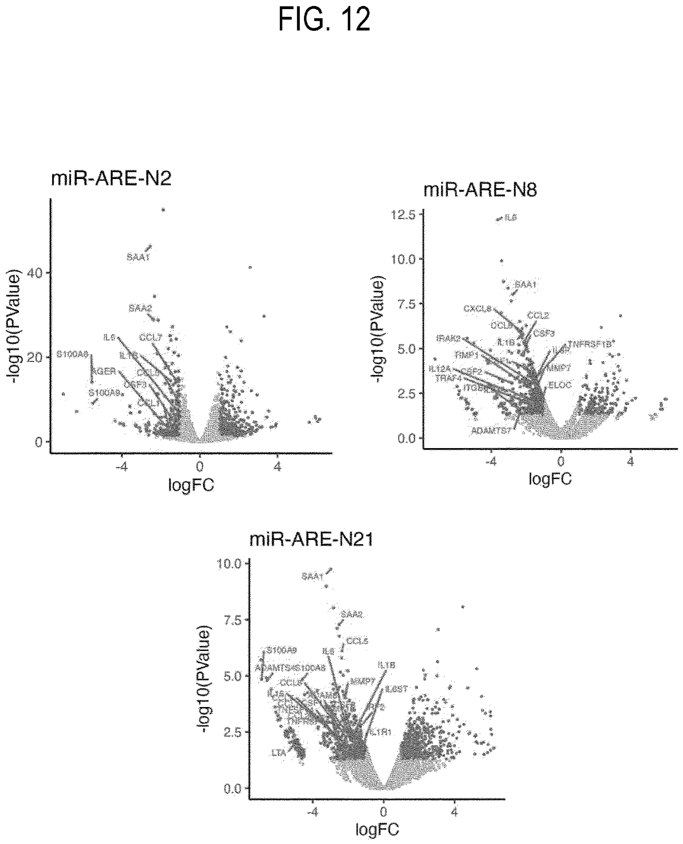

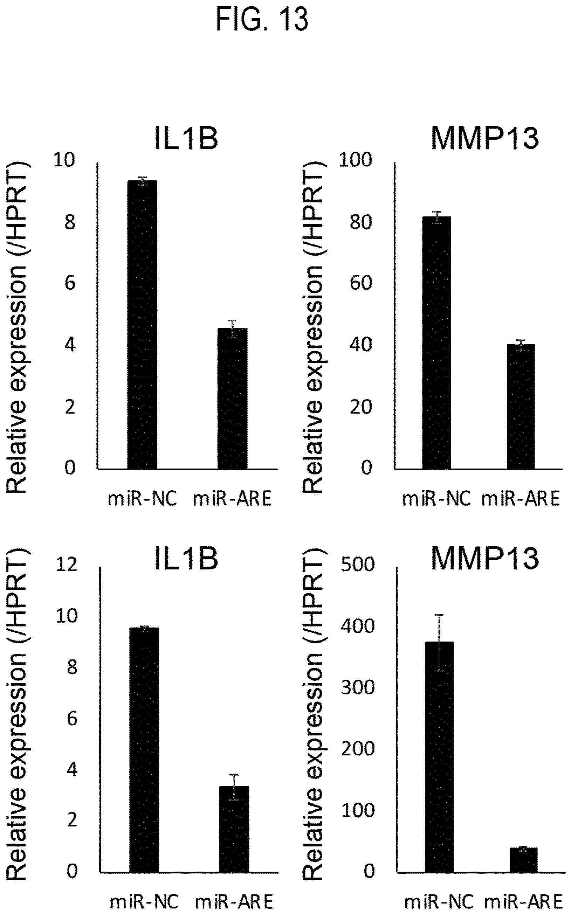

Ionized lipid (DLin-MC3-DMA or SM-102), phospholipid (1,2-DSPC), cholesterol, and PEGylated lipid (DMG-PEG(2000)) were dissolved in ethanol and mixed at a molar ratio of 50:10:38.5:1.5. The lipid solution was mixed with miRNA dissolved in sodium acetate, and then the particle diameter was adjusted by filtration (100 nm). The solution was dialyzed against PBS, and then LNPs were concentrated via ultrafiltration. LNPs containing control miRNA or miR-ARE-N2 (equivalent to 2 µg in terms of base content) were added to SW1353 cells. After 48 h, cells were stimulated with IL-1β (WAKO Chemicals) for 6 h, and IL1B and MMP13 expressions were quantified by real-time PCR using HPRT as a control. As a result, introduction of miR-ARE reduced gene expression of IL-1B and MMP13 (Fig.10). In addition, it was found that use of DLin-MC3-DMA as a ionized lipid showed higher suppression efficiency.

These examples demonstrate that the miRNA constructs of the present invention possess gene expression suppression effects against various inflammation-related genes. Furthermore, the administration of the miRNA constructs of the present invention effectively alleviates inflammatory diseases.

Although preferred embodiments of the present invention are disclosed in this specification, it is apparent to those skilled in the art that these embodiments are provided for illustrative purposes only, and various modifications, alterations, and substitutions can be made without departing from the scope of the present invention. It should be understood that various alternative embodiments described herein can be used when implementing the invention. Furthermore, the contents of all publications, including patents and patent applications referenced in this specification, are hereby incorporated by reference as if expressly stated herein.

### Industrial Applicability

As shown in this specification, the inventors have developed novel artificial miRNA constructs useful for post-transcriptional mRNA regulation, and demonstrated that supplementation of these miRNA constructs can provide a new therapeutic or prophylactic approach for chronic inflammatory diseases. The therapeutic strategy based on artificial miRNAs that do not naturally exist in the body, such as the constructs of the present invention, has been found for the first time by the present inventors. The invention can be extremely useful for the treatment and prevention of chronic inflammatory diseases.

## Claims

1. A miRNA construct having a sequence, the sequence consisting of:
i) a seed sequence recognizing a binding motif of an RNA-binding protein;
ii) an adenine or uracil residue located on the 5' side of the seed sequence; and
iii) a scramble sequence located on the 3' side of the seed sequence, the scramble sequence having a sequence so that a GC content of the entire miRNA construct is 40-60%.

2. The miRNA construct according to Claim 1, wherein the binding motif of the RNA-binding protein is an AU-rich element, a stem-loop motif, a GU-rich element, or a Pumilio-binding motif.

3. The miRNA construct according to Claim 1, wherein the binding motif of the RNA-binding protein is a binding motif of an RNA-binding protein selected from the group consisting of tristetraprolin (TTP), BRF1, BRF2, AUF1, KSRP, Regnase-1, Roquin, CUGBP1, and Pumilio.

4. The miRNA construct according to Claim 1, wherein the miRNA construct has a length of 19 to 25 nucleotides.

5. The miRNA construct according to Claim 1, wherein the binding motif of the RNA-binding protein is a binding motif present in an mRNA of a gene involved in an inflammatory disease and/or cancer.

6. A method for designing a miRNA construct, comprising:
i) the step of selecting a seed sequence recognizing an RNA-binding protein binding motif;
ii) the step of placing an adenine or uracil residue on the 5' side of the seed sequence; and
iii) the step of placing a scramble sequence on the 3' side of the seed sequence, the scramble sequence having a sequence so that a GC content of the entire miRNA construct is 40-60%.

7. A method for screening a miRNA library for a miRNA construct, the method comprising:
the step of producing the miRNA library comprising miRNA constructs, the miRNA constructs having a sequence, the sequence consisting of:
i) a seed sequence recognizing a binding motif of an RNA-binding protein;
ii) an adenine or uracil residue placed on the 5' side of the seed sequence; and
iii) a scramble sequence placed on the 3' side of the seed sequence, the scramble sequence having a sequence so that the GC content of the entire miRNA construct is 40-60%; and
the step of selecting a miRNA construct based on whether the miRNA construct has an effect of suppressing expression of a gene involved in at least one type of inflammatory disease, or an effect of improving inflammation in a subject.

8. A pharmaceutical composition comprising at least one substance selected from the group consisting of the miRNA construct according to Claim 1, a mimic of the miRNA construct according to Claim 1, and DNA encoding the miRNA construct according to Claim 1.

9. The pharmaceutical composition according to Claim 8, wherein the pharmaceutical composition is an anti-inflammatory agent.

10. The pharmaceutical composition according to Claim 8, further comprising a nucleic acid transfection enhancer.

11. The pharmaceutical composition according to Claim 8, wherein the miRNA construct or the mimic of the miRNA construct comprises a modified nucleic acid.

12. The pharmaceutical composition according to Claim 8, wherein the pharmaceutical composition is used for treatment of a chronic inflammatory disease.

13. The pharmaceutical composition according to Claim 8, wherein the pharmaceutical composition is used for suppressing an expression of a gene involved in at least one type of inflammatory disease.
